# EUROPEAN PATENT SPECIFICATION

(11) **EP 1 868 673 B1**
(45) Date of publication and mention of the grant of the patent: **08.06.2016**
(21) Application number: 06754741.4
(22) Date of filing: 13.04.2006
(51) Int. Cl.: A61M 15/00, B65D 75/36, B65B 61/02

(54) **BLISTER PACK**
BLISTERPACKUNG
EMBALLAGE-COQUE

(30) Priority: 15.04.2005 GB 0507710
(43) Date of publication of application: 26.12.2007
(73) Proprietor: Vectura Delivery Devices Limited, Chippenham, Wiltshire SN14 6FH (GB)
(72) Inventor: MELINIOTIS, Andreas Mark, Cambridge, CB4 2QB (GB); EVANS, Peter Alan, Cambridgeshire, CB6 2ST (GB); EASON, Stephen William, Norfolk, IP22 1RX (GB); HARMER, Quentin John, Cambridge, CB5 9NJ (GB)
(74) Representative: Summersell, Richard John
(86) International application number: PCT/EP2006/061606
(87) International publication number: WO 2006/108876

(56) References cited:
- WO-A-01/94098
- DE-A1- 19 613 959
- FR-A- 2 381 611
- US-A- 2 358 246
- US-A- 3 909 582
- US-A- 4 286 712
- US-A- 5 630 308
- US-A- 6 055 794
- US-A- 6 155 423
- US-B1- 6 705 467

## Description

The present invention relates to a blister pack and, in particular, to a strip of blisters that are used to store individual doses of medicament in dry powdered form prior to the sequential inhalation of each dose by a patient using an inhalation device equipped with an indexing and piercing mechanism and in which the strip is pre-loaded or fitted by the patient ready for use. The invention also relates to a method of imparting a line of weakness in a blister pack according to the invention, a device or sealing tool for imparting said line of weakness either during or after manufacture of the blister pack and, an inhalation device containing a strip of blisters according to the invention.

Oral or nasal delivery of a medicament using an inhalation device is a particularly attractive method of drug administration as these devices are relatively easy for a patient to use discreetly and in public. As well as delivering medicament to treat local diseases of the airway and other respiratory problems, they have more recently also been used to deliver drugs to the bloodstream via the lungs thereby avoiding the need for hypodermic injections.

In one type of conventional metered dose inhalation device, the powdered medicament is held in a reservoir within a dispensing device that is operable to measure out and dispense a predetermined amount of powder for each dose. However, these devices suffer from poor dose metering capability especially when the size of the dose is relatively small as it is difficult to accurately measure out small amounts of dry powder in such a device. It is also difficult to protect the drug from the ingress of moisture and to seal it from the atmosphere until it is required for administration to a patient.

In view of the foregoing, it has become common for dry powder formulations to be pre-packaged in individual doses, usually in the form of capsules or blisters that each contain a single dose of the powder which has been accurately and consistently measured. A foil blister is preferred over capsules as each dose is protected from the ingress of water and penetration of gases such as oxygen in addition to being shielded from light and UV radiation all of which can have a detrimental effect on the delivery characteristics of the inhaler if a dose becomes exposed to them.

Inhalation devices that receive a blister pack or strip of blisters are known. Actuation of the device causes a mechanism to index and pierce a blister so that when the patient inhales, air is drawn through the blister entraining the dose, which is then carried out of the blister through the device and via the patient's airway down into the lungs.

A blister pack generally comprises a base having a number of spaced apart cavities defining blisters to receive individual doses of medicament and, a lid in the form of a generally planar sheet that is sealed to the base except in the region of the cavities using a sealing tool which compresses the base and lid material together in a region surrounding each cavity. The tool is heated so that the lid is sealed to the base during the compression step. The base material is typically a laminate comprising a polymer layer in contact with the drug, a soft tempered aluminium layer and an external polymer layer. The aluminium provides the moisture and oxygen barrier, whilst the polymer aids the adherence of the foil to the heat seal lacquer and provides a relatively inert layer in contact with the drug. Soft tempered aluminium is ductile so that it can be "cold formed" into a blister shape. It is typically 45µm thick. The outer polymer layer provides additional strength and toughness to the laminate.

The lid material is typically a laminate comprising a heat seal lacquer, a hard rolled aluminium layer (typically 20-30µm thick) and an external lacquer layer. The heat seal lacquer bonds to the polymer layer of the base foil laminate during heat-sealing to provide a seal around the top of the blister cavity. The aluminium layer is hard rolled to facilitate piercing of the blister by the inhalation device when access to the medicament contained therein is required. Materials for the polymer layer in contact with the drug include poly vinyl chloride (PVC), polypropylene (PP) and polyethylene (PE). In the case of PE, the heat seal lacquer on the foil lid is replaced with a further layer of PE. On heat sealing, the two layers of PE melt and weld to each other. The external polymer layer on the base foil is typically oriented polyamide (oPA). The polymer layer in contact with the drug is typically PVC of 60µm thickness. However, a thinner layer of 30µm or 15µm may be used, for example, where a more flexible laminate is required.

It will be appreciated that different types of medicament possess varying degrees of sensitivity to various environmental influences and so a foil blister of the type described above provides good environmental protection for the medicament and protects it against moisture ingress, oxygen and other gases. The foil conveniently also protects the drug from light. Although the foil material itself is impermeable to moisture and gases, providing it is not punctured, the polymer layers are permeable to a greater or lesser extent. The permeability is typically defined by a moisture or gas transmission rate over a given time. The transmission rate depends on the type of material, the thickness of the permeable layer and distance of the transmission path. Thus the level of protection provided is determined in part by the breadth of the seal around the blister as this determines the distance any moisture or oxygen has to travel through the polymer layer from the edge of the foil laminate to the blister cavity.

In a strip of blisters, ingress can occur from the edges of the strip or from an adjacent blister that has been punctured. Thus the required breadth of seal should be maintained both from the blister cavity to the edges of the strip and from one blister cavity to an adjacent blister cavity. This distance between the blister cavities or seal breadth should be at least 2mm although at least 2.5mm is more preferable when the medicament is not particularly sensitive to environmental factors. However, a greater distance such as 3, 4 or 5mm or more will afford improved environmental protection and should be used when the medicament is more sensitive to environmental factors.

It is desirable for an inhalation device, such as those used to treat a respiratory disease such as asthma or COPD, to be able to contain sufficient doses for at least one month's treatment. Typically, this requires an inhaler with 30 blisters (for a once daily dose) or 60 blisters (for a twice daily dose). It is known from GB2242134 to provide a device that is capable of receiving an individually sealed foil blister strip of 60 doses in which the lid is peeled away from the base of the strip by the device to enable access to the dose to be obtained. However, the device disclosed in this document is provided with chambers to receive both the used blister base and the lid that has been peeled away from the base and this makes the device unnecessarily large.

An alternative approach is to facilitate the detachment of used blisters from the unused blisters that remain in the strip so that the used blisters may be discarded. This allows the device to be smaller as there is no longer any requirement to store used blisters in the device.

A problem with detaching used blisters is that the external and internal polymer layers on the base foil laminate make it tough and difficult to tear. It is therefore known, for example from EP0469814A, to provide the strip with a series of perforations in the foil between blisters to facilitate their separation by tearing along the perforations. However, when a strip is provided with perforations, the distance between blisters has to be increased and maybe even doubled because the foil is cut by the perforating process thereby creating a break in the moisture seal. Increasing the distance between adjacent blisters increases the sealing distance, i.e. the distance moisture or gas has to travel to reach the drug, and so restores the environmental protection to a similar level found in a blister strip that is not provided with perforations. However, a disadvantage with increasing the distance between adjacent blisters is that the resulting blister strip is considerably longer and so a larger device is required to contain them. Furthermore, in a device that is equipped with an indexing mechanism for incrementally advancing the blisters to a piercing position, an increase in the distance between blisters requires a greater incremental movement to advance the blister strip leading to an increase in the complexity or size of the indexing mechanism.

The present invention seeks to provide a blister pack which is tearable but overcomes or substantially alleviates the problems associated with a perforated strip. In particular the invention seeks to provide a strip which facilitates easy separation of used blisters from those that remain and enables the minimum distance between blisters to be maintained without compromising the integrity of the seal between the blisters and the environmental protection provided by the seal.

According to the present invention, there is provided a blister pack according to claim 1. pThe package includes an inner polymer layer on the foil and in one embodiment

region of weakness is formed by substantially displacing the inner polymer layer, in addition to substantially removing or displacing the outer polymer layer, from the foil.

The blister pack comprises a base portion in which the blister cavities are formed and a substantially planar lid portion sealing the blister cavities.

The blister package is in the form of an elongate strip of blisters and, preferably, a region of weakness is provided between each blister of the strip. Advantageously, each region of weakness is in the form of a straight narrow strip or line and extends across the strip substantially at right angles with respect to the longitudinal edges of the strip.

The strip is sufficiently flexible to enable it to be wound into a roll for insertion into an inhalation device equipped with an indexing mechanism for advancing the blisters one at a time to a piercing station to enable the dose contained therein to be accessed and inhaled by a patient

Although the region of weakness may be unbroken, it is also envisaged that, in one embodiment, one or more regions of weakness may be discontinuous. In this arrangement, the outer polymer layer, and possibly the inner polymer layer, are substantially removed or displaced from discrete, spaced apart regions extending along each region of weakness so that the region of weakness is formed from a series of weakened and non-weakened sections.

In a preferred embodiment, a region of weakness may be provided at an edge of a pack to facilitate the initiation of a tear As the force required to initiate a tear is greater than the force required to continue tearing once a tear has been initiated, a region of weakness at the edge of the pack is alone sufficient to enable separation.

In one embodiment, an edge of the pack may include a notch or a nick or a perforation or a region which has been highly compressed, scored or impacted or a region that has been heated and compressed or otherwise weakened to facilitate the initiation of a tear.

The blister package comprises a lid and a base wherein the blisters are formed in the base, the region of weakness also being formed in the base.

In an alternative embodiment, regions of weakness are formed by locally melting or ablating or otherwise weakening the outer polymer layer. In a preferred embodiment a laser locally melts, ablates or softens or otherwise weakens the outer polymer layer.

In alternative embodiments regions of weakness may be formed by scoring with a rotary or straight blade (often called "kiss cutting") or mechanically forming by nipping between two edges or local impact or by local pressure.

If the blister package is provided with means for initiating a tear where the regions of weakness meet the edge of the package to facilitate the start of a tear along the regions of weakness, these can be formed by any suitable means including kiss cutting, perforation, die cutting, application of a hot tool, application of pressure or laser ablation.

In a preferred embodiment, at least a portion of the region of weakness is a narrow strip or line. The region of weakness may also include an enlarged region where the line meets an edge of the pack.. In one particular embodiment, the narrow strip or line extends across the pack between two enlarged regions where the ends of the line meet the edges of the pack.

According to the present invention, there is also provided a method of imparting a region of weakness in a blister pack between adjacent blister cavities of the pack according to claim 14.

The step of substantially removing or displacing a portion of the outer polymer layer from the foil layer to form said region of weakness preferably includes the step of applying heat and pressure to the pack to soften or melt a portion of the outer polymer layer and compress and/or push said portion away from the foil in said region.

In one embodiment, the method includes the step of cutting a portion of the pack in a region where a region of weakness meets an edge of the pack to form means for initiating a tear in the region of weakness.

The present invention also provides an inhalation device containing a blister pack according to the invention.

Although the blister package of the present invention is intended for use in many different devices, it is primarily intended for use in the inhalation device disclosed in the Applicant's co-pending international PCT application no. PCT/GB2004/004416 published as WO2005/037353 A1, which includes an actuator for indexing and piercing each of the blisters and in which used blisters protrude from the housing to facilitate their removal from those unused blisters that remain in the housing.

In the aforementioned device, a strip of blisters is coiled inside the device. However, it has been established that coiling the strip can cause the lidding foil to come under excessive stress in the vicinity of the regions of weakness. This is caused by the thickness of the laminate and the concentration of stress due to the thinning of the laminate at the region of weakness. These problems are mitigated by using a cold formed foil with a thinner polymer layer in contact with the drug. The polymer layer in contact with the drug is preferably less than 60µm and, most preferably, between 15µm and 40µm thick.. In a particularly preferred embodiment, the thickness of the polymer layer is 30µm. Not only does a thinner polymer layer reduce the stress applied to the lidding foil when the strip is coiled, but a thinner polymer layer also makes the laminate much easier to tear, especially once a tear has been initiated.

Embodiments of the present invention will now be described, by way of example only, with reference to the accompanying drawings, in which:
FIGURE 1 illustrates a side-sectional view through a portion of a strip of blisters showing two adjacent blisters prior to processing of the strip to form a line of weakness between them, the thickness of the material layers from which the strip is formed being shown grossly over exaggerated to facilitate understanding of its construction;
FIGURE 2 illustrates a portion of the cross sectional view of Figure 1 between the lines A-A and B-B shown in Figure 1 and after a line of weakness between adjacent blisters has been formed;
FIGURE 3 illustrates a simplified perspective view of a tool that is used to form the lines of weakness between blisters and shows a strip of blisters in which three lines of weakness have already been formed;
FIGURES 4A to 4D illustrate alternative cross-sections through the blade member of the tool shown in Figure 3;
FIGURE 5 illustrates a side sectional view of the blade member of the tool shown in Figure 3 but in which the blade member is provided with stops to control the position of the blade;
FIGURE 6 illustrates a side sectional view of the blade member of the tool shown in Figure 3 but in which the blade member is provided with cutting elements on each edge to cut a notch in the blister strip at either end of the line of weakness formed by the blade member;
FIGURE 7 illustrates a perspective view of a blister strip which has in which three lines of weakness have been formed between adjacent blisters and notches have been cut at each end of each line of weakness, using the blade member shown in Figure 6;
FIGURE 8 illustrates an alternative tool for forming the lines of weakness between blisters;
FIGURE 9A and 9B illustrate a perspective view of a strip of blisters and, an enlarged perspective view of a line of weakness extending between adjacent blisters respectively, and which have been formed using the alternative tool illustrated in Figure 8; and
FIGURES 10A, 10B and 10C illustrate three different versions of a portion of a blister sealing tool which forms regions to facilitate the initiation and/or propagation of a tear between blister cavities.

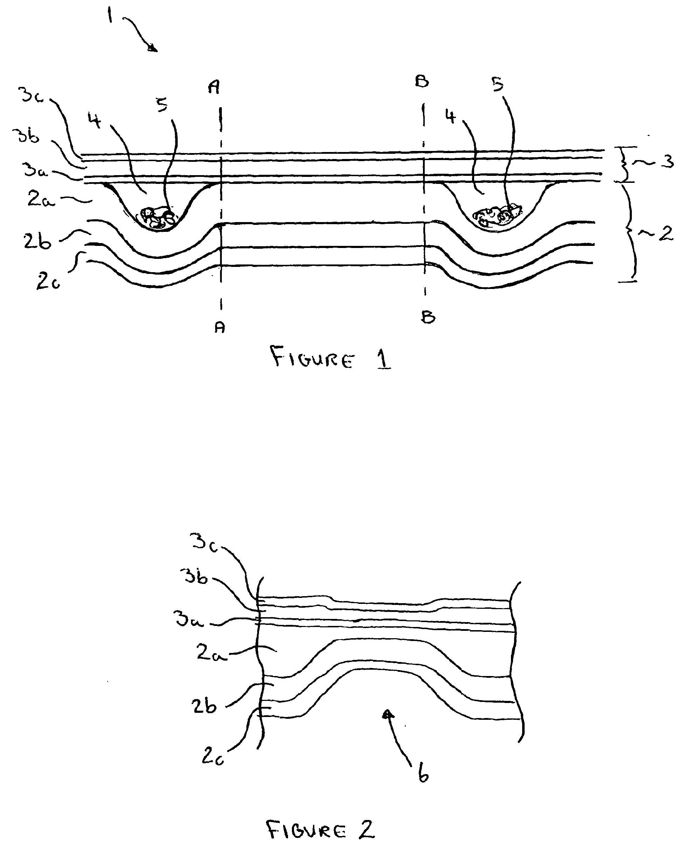

Referring now to the drawings, there is shown in Figure 1, a cross section through a portion of a strip of blisters 1 showing two blisters 4 and a portion of the strip that extends between them and in which a line of weakness 6 is to be formed. Although the invention is described with reference to a elongate strip 1 of blisters which are sufficiently flexible to enable them to be coiled for insertion into an inhalation device, such as the device disclosed in the Applicant's co-pending GB application no. 0324358.1,

The blister strip 1 shown in Figure 1 comprises a base portion 2 and a lid portion 3. The base portion 2, in which the blister cavities 4 each containing a dose of medicament 5 are formed, is a laminate of three layers: a polymer layer 2a which contacts the medicament, a soft tempered aluminium foil layer 2b and an external polymer layer 2c. The lid portion 3 is a planar laminate sheet formed from three layers: a heat seal lacquer layer 3a which is bonded to the polymer layer 2a of the base portion 2 during heat sealing to provide a seal around the top of the blister cavity 4, an aluminium foil layer 3b and an external lacquer layer 3c. The specific materials and constructional aspects of the blister pack 1 have already been described above and so will not be repeated again.

In a modified blister strip 1, the base portion 2 may include an additional polymer layer (not shown) on the side away from the blister cavity 4 to create a more symmetric laminate that is less susceptible to warping or distortion during the cold forming of the blister cavities 4.

Figure 2 shows a portion of the blister strip 1 between the lines A-A and B-B shown in Figure 1, after a line of weakness 6 has been formed therein between adjacent blister cavities 4. It will be appreciated that the thickness of the laminates in the region of the line of weakness 6 have been greatly reduced and compressed and that some of external polymer layer 2c of the base portion 2 has been displaced to reduce its thickness, although it is also envisaged that a portion of the external polymer layer 2 may be removed altogether. An important aspect of the invention is that the foil layer 2b,3b of the base portion 2 and of the lid portion 3 remains undamaged and unbroken despite the formation of the line of weakness 6.

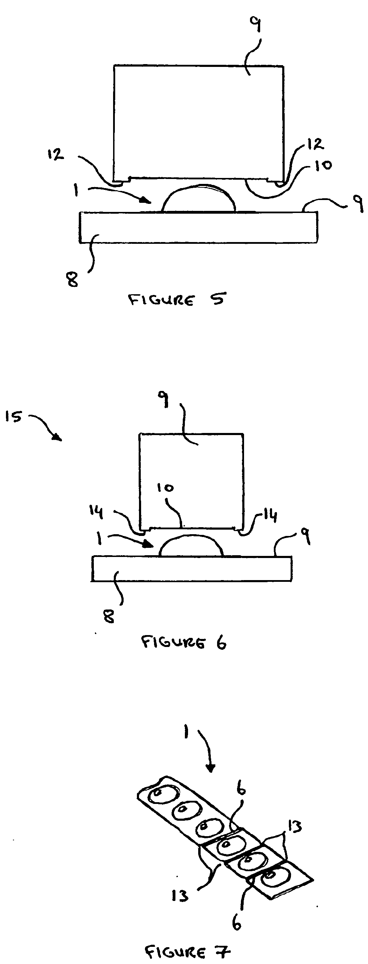

As the force required to initiate a tear in a blister strip 1 at a line of weakness 6 is greater than the force required to continue tearing once a tear has been initiated, initiating features 13 may be provided at one or both of the edges of each line of weakness 6 to facilitate the initiation of a tear. The initiator 13 may be a notch, a nick or a perforation or a region which has been highly compressed, scored or impacted or a region that has been heated and compressed or otherwise weakened. A strip 1 of blisters in which each line of weakness 6 is provided with a tear initiator 13 is illustrated in Figure 7. It will be appreciated that a initiator 13, such as a cut, at the edge of the strip 1 between the blisters 4 does not affect the minimum seal breadth from the edge of the strip 1 to the blister cavity 4 providing that the initiator 13 is outside a region defined by one cavity 4 to cavity 4 distance from the perimeter of the cavity 4.

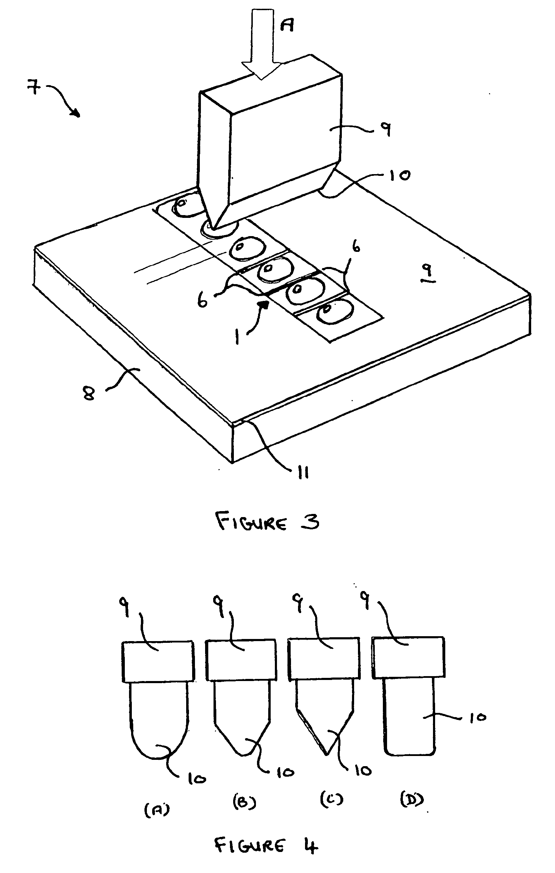

Tools for, and methods of, forming the line of weakness 6 shown in the blister strip 1 of Figure 2 will now be described with reference to Figures 3 to 9.

Figure 3 shows a tool 7 comprising a table 8 having an upper surface 9 and a heated blade member 9 disposed above the table 8 which is provided with a mechanism (not shown) for reciprocating the blade member 9 towards and away from the upper surface of the table 8 in the direction indicated by arrow "A". The heated blade member 9 includes a tool tip 10 for engagement with a blister strip 1 located on the upper surface of the table 8. A predetermined pressure is applied to the blister strip 1 by the blade member 9 when it moves towards and into contact with a blister strip located on the table 8 to compress the blister strip and the heat causes the external polymer layer 2c to melt and soften causing it to be partially or wholly displaced in a region where the blade member 9 contacts the blister strip 1. Although it is intended that the blister strip 1 is placed face-down on the table 8, i.e. with its lid portion 3 against the upper surface 9 of the table 8 so that the tool tip 10 engages and forms the line of weakness in the base portion 2, it will be appreciated that the blister strip 1 may also be placed the other way up on the table 8 so that the tool tip engages the lid portion 3 of the strip 1.

Suitable materials for the blade member 9 include aluminium and aluminium alloys, preferably hard anodised, and stainless steels. Advantageously the blade member 9 may be coated with a low friction or "non-stick" coating such as PTFE (polytetraflourethylene) to help ensure that the outer polymer layer 2c on the base portion 2 does not adhere to the blade member 9 during compression and heating, as material adhering to the blade member 9 would reduce the effectiveness of the line of weakness formation.

If the blister strip 1 is modified by the presence of an additional polymer layer (not shown), this additional layer can help reduce the propensity of the foil layer 2b to adhere to the blade member 9, especially if the additional polymer layer is formed from a material that is less susceptible to "stringiness" when it softens, for example PVC.

The tool tip 10 of the blade member may have a radius of 0.2 to 1.0mm and more preferably 0.4 to 0.6mm. In a particularly preferred embodiment, the radius of the tool tip 10 is 0.5mm. A cross-section through the tool tip 10 is shown in Figure 4A. However, it is also envisaged that the tool tip 10 may take alternative configurations to include a V-shaped blade with a radius at the tip (Figure 4B), a V-shaped blade with no radius at the tip (Figure 4C) or a flat blade (Figure 4D).

The table 8 may be formed from aluminium and/or aluminium alloys, preferably hard anodised, stainless steels and high temperature polymers such as PEEK (poly ether ether ketone), polyamide or PTFE. Where required, for example in the case where the medicament 5 is sensitive to temperature, the table 8 may be cooled.

The upper surface 9 of the table 8 may optionally be provided with a thin resilient layer 11. Layer 11 assists in the formation of the line of weakness 6 by reducing the sensitivity of the process to the level of force applied and allows the foil layers 2b,3b of the blister strip 1 to bend slightly during forming so that the stresses, particularly shear and tearing stresses, induced in the foil layers 2b,3b are reduced thereby ensuring that they are not broken or cut by high levels of force. Suitable materials for the resilient layer 11 include polyamides, polyimides, PTFE, ETFE and silicone rubbers. The layer 11 is preferably less than 1mm thick and more preferably less than 0.5mm. In a preferred embodiment the resilient layer 11 is formed from a 0.3mm layer of polyamide.

To ensure that the outer and inner polymer layers 2a,2c are softened sufficiently and so that the material of the outer polymer layer 2c is squeezed to the sides of the blade member 9 without cutting the foil layers 2b,3b, it is important to carefully select the temperature of the blade member 9 and the duration of contact with the blister pack 1. Suitable and preferred ranges for the key operating parameters are shown in the table below. It will be clear to those skilled in the art that the parameters interact with each other, for example increasing the duration will allow a lower force to be applied and increasing temperature may allow a shorter duration to be employed.

| | Suitable range | Preferred range |
|---|---|---|
| Top tool temperature (°C) | 230 - 280 | 235 - 245 |
| Force per tear line (N) | 300 - 600 | 400 - 500 |
| Duration (s) | 0.1 - 3.0 | 0.5 - 1.0 |

The application of heat and pressure needs to be controlled to achieve a repeatable line of weakness 6. One option is to control the force or pressure applied by, for example, using a spring at a predefined level of compression or a pneumatic cylinder at a predetermined pressure to provide the force. Alternatively, the blade member 9 may be provided with one or more stop members 12 which holds the tool tip 10 a predetermined distance from the upper surface 9 of the table 8 so that the blister strip 1 is compressed only by a predetermined amount by the blade member 9. In the embodiment illustrated in figure 5, stop members 12 are provided on each edge of the tool tip 10. The distance between the tool tip 10 and the upper surface 9 of the table 8 may be adjustable to enable lines of weakness 6 to be formed in blister strips 1 having various thicknesses of laminate. Preferably the distance between the tool tip 10 and the upper surface 9 is selected to be between 25% and 100% of the total thickness of the laminate.

If a tear initiator 13 is to be provided, this can be formed in the same operation as the formation of the line of weakness 6. Figure 6 shows an alternative tool 15 for forming a line of weakness 6 together with notches at each end of the line of weakness 6 to facilitate the initiation of a tear. Cutting elements 14 depend from the heated tool member 9 and are received in mating recesses (not shown) cut into the upper surface 9 of the table 8. The cutting elements 14 cut a notch in the blister pack 1 as the tool tip 10 forms a line of weakening in the strip 1.

An effective line of weakness 6 need not be continuous. For example, one or more unweakened regions may be left in a line of weakness 6 in order to maintain the tensile strength of the strip 1 to, for example, facilitate handling during manufacture and indexing of the strip 1 in an inhalation device and to prevent accidental tearing of the strip 1. A line of weakness 6 may therefore comprise regions that have been weakened and regions that have not been weakened or regions with differing levels of weakening.

It will be appreciated that more than one line of weakness 6 can be formed at one time by applying the weakening at a plurality of points simultaneously using for example, a tool with multiple blade members 9. Preferably, lines of weakness 6 are formed along a substantial portion or the whole of the strip 1 in a single operation. Alternatively they may be formed in a continuous process. Means for creating intermittent or continuous processes for high volume manufacture are well known in the field of blister processing machinery. Similarly, more than one strip can be processed at a time by processing strips side by side simultaneously.

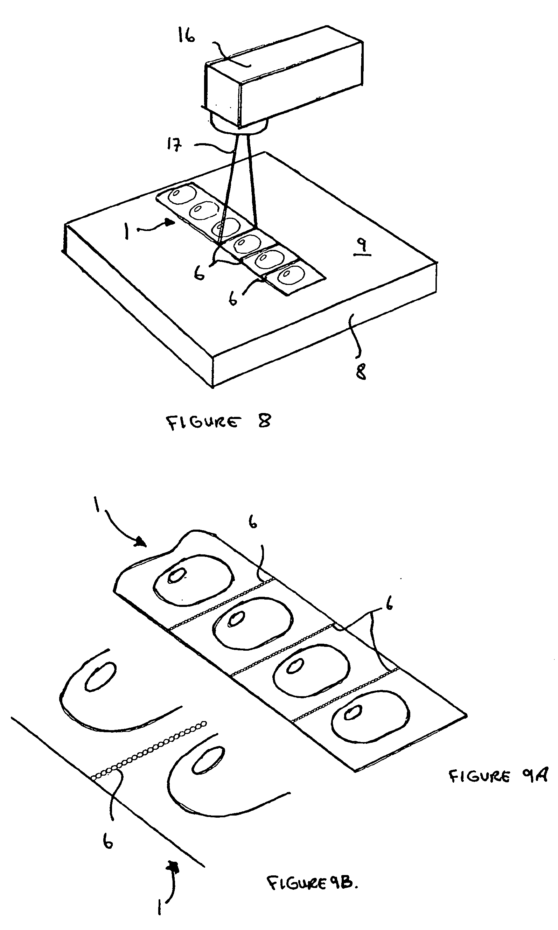

Figure 8 illustrates a further embodiment in which a laser 16 is used to form lines of weakness in the strip 1. According to this embodiment a laser beam 17 emitted by the laser 16 scans across the strip 1 in a predetermined pattern and locally modifies the polymer layer 2c by melting, ablation or a combination of the two, to form a line of weakness 6 without affecting the foil layer 2b. The laser 16 may be configured to ablate only a proportion of the thickness of the polymer layer 2c although controlling the accuracy of the depth of the ablation may be difficult in a continuous process where the foil layer 2b is subject to movement. Therefore, it is preferable if the laser 16 ablates the polymer layer 2c to its full depth. The laser 16 may ablate the ploymer layer 2c across only a portion of the width of the strip 1. For example in a dotted or dot matrix or dashed pattern such that along the line of weakness 6 there are alternate regions of ablated and unablated polymer, as shown in Figures 9A and 9B. In this way the strip can be made tearable without weakening it unduly.

The laser 16 may be a CO₂ laser or a YAG laser but is preferably a CO₂ laser. The type and power of the laser 16 is chosen to give effective ablation of the polymer layer 2c without damaging the aluminium foil layer 2b below. Forming a line of weakness with a laser can be combined with any of the aforementioned methods for initiating a tear including a nick, notch or kiss cut.

Advantageously for high volume production the process of making the blister strips 1 is continuous or comprises a combination of continuous and intermittent stations depending on the type of operation. For example, cold forming of a blister shape is often carried out by an intermittent process. In a continuous process, the laser 16 is programmed to scan across the foil layer 2b to form a line of weakness 6 and then index to the next position in synchronisation with the indexing of the strip 1 through the process. This may be achieved by scanning the laser beam 17 or by moving the strip 1. In an intermittent process the laser 16 may form a number of lines of weakness by scanning the beam 17 before the strip 1 is indexed along by a number of blisters 4.

It will be appreciated that all of the aforementioned embodiments address a method and apparatus for forming a region of weakness in a preformed blister strip. However, it is also envisaged that the formation of a region of weakness between blisters can be formed simultaneously with the manufacture of the blister i.e. at the same time as when the lid is sealed to the base.

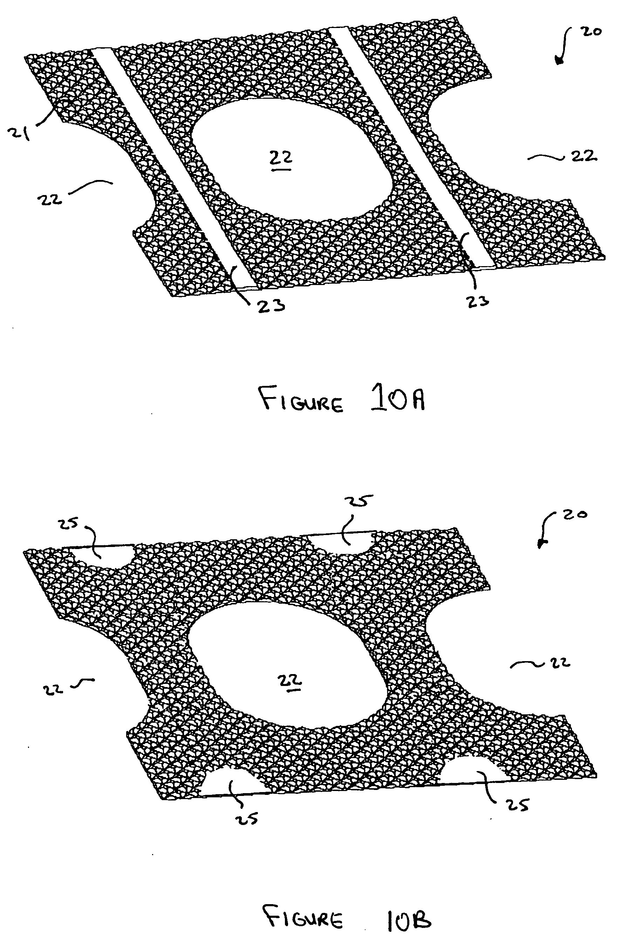

Figure 10A illustrates a portion of a sealing tool or plate 20 comprising a knurled or otherwise roughened patterned surface 21 that contacts the lidding material and performs the function of sealing it onto the base. The sealing tool 20 has an aperture 22 corresponding to a blister cavity so that the lidding material is unaffected by the tool 20 in the region of the lidding material that extends over the cavity. On either side of the aperture 22 is a raised area 23 for creating a tear propagation line of weakness in the strip on either side of a blister cavity. In a preferred embodiment, the raised area is substantially level with the tips or topmost surfaces of the knurling pattern which forms the seal. The raised areas 23 creates a region between the blisters which is both slightly thinned and stiffened as the laminate is squeezed against the sealing tool which is heated so as to cause the sealing lacquer to melt This region is therefore slightly more brittle and easily torn once a tear is initiated.

It will be appreciated that the sealing tool may be applied to either side of the blister strip to seal the lidding material to the base. Furthermore, the lid and base material may be squeezed between two similar sealing tools.

Figure 10B illustrates a modified version of a portion of the sealing tool shown in Figure 10A. In this embodiment, the raised areas 25 take the form of enlarged regions at the edge of the strip between the apertures 22 to create tear initiation regions that facilitate the initiation of a tear. In a preferred embodiment, the raised region is substantially level with the tips or topmost surfaces of the kurled pattern which forms the seal. Alternatively, either of the raised areas shown in Figures 10A and 10B may be slightly proud of the knurled pattern by up to 0.2mm. The knurled pattern is typically between 0.025mm and 0.2mm in depth and preferably between 0.04mm and 0.1mm.

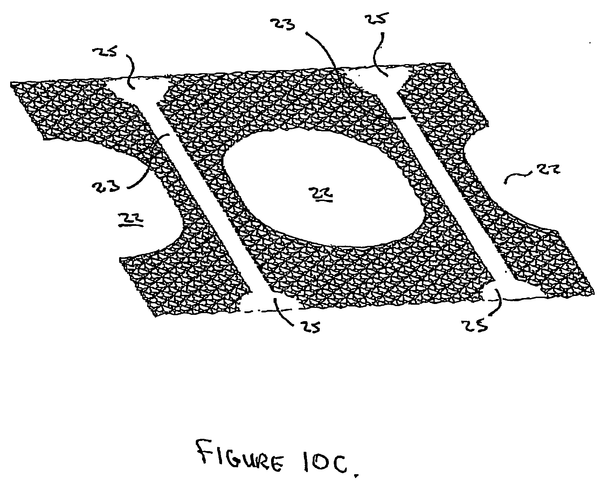

Figure 10C illustrates yet another modified version of a portion of the sealing tool shown in Figures 10A and 10B. In this version, the raised areas 23 as shown in Figure 10A are combined with the raised areas 25 as shown in Figure 10B. The combined raised region 23,25 is used to both initiate and propagate a tear. The tear initiation regions 25 may be slightly higher than the propagation regions 23 as the laminate needs to be weaker in the region of initiation.

Many modifications and variations of the invention falling within the terms of the following claims will be apparent to those skilled in the art and the foregoing description should be regarded as a description of the preferred embodiments of the invention only.

## Claims

1. A blister pack in the form of an elongate strip which is sufficiently flexible to enable it to be wound into a roll for insertion into an inhalation device comprising a plurality of spaced blister cavities (4) which receive and store an individual dose of powdered medicament for inhalation by a user, wherein the pack includes a base (2) in which the blister cavities (4) are formed and a substantially planar lid portion (3) sealing the blister cavities (4), the base (2) and the lid portion (3) each comprising a laminate, the base laminate including a foil layer (2b) which is impermeable to moisture and gases, an outer polymer layer (2c) and an inner polymer layer (2a) on the opposite side of the foil layer (2b) to the outer polymer layer (2c), **characterised by** a region of weakness (6) formed by substantially removing a portion of the outer polymer layer (2c) from the base laminate, or by thinning a portion of said outer polymer layer (2c) of the base laminate, between each blister cavity (4), or a number of blister cavities (4), without damaging the foil layer (2b) of said base laminate.

2. A blister pack according to claim 1, wherein the region of weakness (6) is formed by substantially displacing the inner polymer layer (2a), in addition to substantially removing or displacing the outer polymer layer (2c), from the base laminate.

3. A blister pack according to claim 2, wherein the inner polymer layer (2a) has a thickness which is less than 60µm.

4. A blister pack according to claim 3, wherein the inner polymer layer (2a) has a thickness of between 15µm and 40µm.

5. A blister pack according to claim 4, wherein the inner polymer layer (2a) has a thickness of 30µm.

6. A blister pack according to any preceding claim, wherein the region of weakness (6) is provided between each blister of the strip.

7. A blister pack according to any preceding claim, wherein the or each region of weakness (6) is discontinuous.

8. A blister pack according to claim 7, wherein the outer polymer layer (2c) is substantially removed or displaced from discrete, spaced apart regions extending along each region of weakness (6) so that each region of weakness is formed from a series of weakened and non-weakened sections.

9. A blister pack according to any preceding claim, wherein the region of weakness (6) is formed at an edge of a pack to facilitate the initiation of a tear.

10. A blister pack according to any preceding claim, comprising a notch, nick or perforation (13) in the edge of the pack to facilitate initiation of a tear.

11. A blister pack according to any preceding claim, wherein at least a portion of the region of weakness (6) is a narrow strip or line.

12. A blister pack according to claim 11, wherein the region of weakness (6) comprises a narrow strip or line and an enlarged region of weakness where the line meets an edge of the pack.

13. A blister pack according to claim 12, wherein the narrow strip or line extends across the pack between two enlarged regions of weakness where the ends of the line meet the edges of the pack.

14. A method of imparting a region of weakness in a blister pack between adjacent blister cavities (4) of the pack which receive and store individual doses of powdered medicament for inhalation by a user, the pack being in the form of an elongate strip (1) which is sufficiently flexible to enable it to be wound into a roll for insertion into an inhalation device and including a base (2) in which the blister cavities (4) are formed and a substantially planar lid portion (3) sealing the blister cavities (4), the base (2) and the lid portion (3) each comprising a laminate including a foil layer (2b,3b) which is impermeable to moisture and gases, an outer polymer layer (2c, 3c) and an inner polymer layer (2a, 3a) on the opposite side of the foil layer (2b,3b) to the outer polymer layer (2c, 3c), wherein the method includes the step of substantially removing a portion of the outer polymer layer (2c, 3c) from the base or lid portion or thinning a portion of said outer polymer layer (2c, 3c) of the base or lid portion, without damaging the foil layer (2b,3b) of the laminate, to form said region of weakness.

15. A method according to claim 14, wherein the step of substantially removing or displacing a portion of the outer polymer layer (2c) from the base laminate to form said region of weakness (6) includes the step of applying heat to the pack to soften or melt a portion of the outer polymer layer (2c) from the foil layer (2b) in said region.

16. A method according to claim 15, including the step of forming means for initiating a tear (13) where the region of weakness (6) meets an edge of the pack.

17. A method according to claim 15 or 16, wherein the step of forming said region of weakness (6) by applying heat to the pack to soften or melt a portion of the outer polymer layer (2c) without damaging the foil layer (2b) comprises the step of using a laser.

18. An inhalation device containing a blister pack according to any of claims 1 to 13.

## Patentansprüche

1. Blisterpackung in der Form eines länglichen Streifens, der so flexibel ist, dass er zu einer Rolle aufgewickelt werden kann, um in eine Inhalationsvorrichtung eingeführt zu werden, die eine Vielzahl von beabstandeten Blisterhohlräumen (4) umfasst, in denen eine Einzeldosis pulverförmigen Medikaments zur Inhalierung durch einen Benutzer aufgenommen und gelagert ist, wobei die Packung eine Basis (2), in der die Blisterhohlräume (4) ausgebildet sind, und einen im Wesentlichen planaren Deckelabschnitt (3) aufweist, der die Blisterhohlräume (4) versiegelt, wobei die Basis (2) und der Deckelabschnitt (3) jeweils ein Laminat umfassen, wobei das Basislaminat eine Folienschicht (2b, 3b), die feuchtigkeits- und gasundurchlässig ist, eine äußere Polymerschicht (2c) und eine innere Polymerschicht (2a) aufweist, die sich auf der der äußeren Polymerschicht (2c) gegenüberliegenden Seite der Folienschicht (2b, 3b) befindet, **gekennzeichnet durch** einen Schwächebereich (6), der **dadurch** gebildet ist, dass ein Abschnitt der äußeren Polymerschicht (2c) vom Basislaminat im Wesentlichen entfernt wird, oder **durch** Verdünnen eines Abschnitts der äußeren Polymerschicht (2c) des Basislaminats zwischen jedem Blisterhohlraum (4) oder mehreren Blisterhohlräumen (4) ohne Beschädigung der Folienschicht (2b, 3b) des Basislaminats.

2. Blisterpackung nach Anspruch 1, wobei der Schwächebereich (6) dadurch gebildet ist, dass die innere Polymerschicht (2a) im Wesentlichen verschoben wird, und zusätzlich, dass die äußere Polymerschicht (2c) vom Basislaminat im Wesentlichen entfernt oder verschoben wird.

3. Blisterpackung nach Anspruch 2, wobei die innere Polymerschicht (2a) eine Dicke von weniger als 60 µm hat.

4. Blisterpackung nach Anspruch 3, wobei die innere Polymerschicht (2a) eine Dicke zwischen 15 µm und 40 µm hat.

5. Blisterpackung nach Anspruch 4, wobei die innere Polymerschicht (2a) eine Dicke von 30 µm hat.

6. Blisterpackung nach einem der vorhergehenden Ansprüche, wobei der Schwächebereich (6) zwischen jedem Blister des Streifens vorgesehen ist.

7. Blisterpackung nach einem der vorhergehenden Ansprüche, wobei der oder jeder Schwächebereich (6) diskontinuierlich ist.

8. Blisterpackung nach Anspruch 7, wobei die äußere Polymerschicht (2c) von einzelnen, beabstandeten Bereichen, die sich entlang jedem Schwächebereich (6) erstrecken, im Wesentlichen entfernt oder verschoben ist, so dass jeder Schwächebereich aus einer Reihe von geschwächten und nicht geschwächten Sektionen gebildet ist.

9. Blisterpackung nach einem der vorhergehenden Ansprüche, wobei der Schwächebereich (6) zur Erleichterung eines Anreißens an einem Rand einer Packung gebildet ist.

10. Blisterpackung nach einem der vorhergehenden Ansprüche, umfassend eine Kerbe, Einkerbung oder Perforation (13) im Rand der Packung zur Erleichterung eines Anreißens.

11. Blisterpackung nach einem der vorhergehenden Ansprüche, wobei mindestens ein Abschnitt des Schwächebereichs (6) ein schmaler Streifen oder eine Linie ist.

12. Blisterpackung nach Anspruch 11, wobei der Schwächebereich (6) einen schmalen Streifen oder eine Linie und dort, wo die Linie auf einen Rand der Packung trifft, einen verbreiterten Schwächebereich umfasst.

13. Blisterpackung nach Anspruch 12, wobei sich der schmale Streifen oder die Linie zwischen zwei verbreiterten Schwächebereichen, dort, wo die Enden der Linie auf die Ränder der Packung treffen, über die Packung erstreckt.

14. Verfahren zum Versehen einer Blisterpackung mit einem Schwächebereich zwischen benachbarten Blisterhohlräumen (4) der Packung, in denen Einzeldosen pulverförmigen Medikaments zur Inhalierung durch einen Benutzer aufgenommen und gelagert sind, wobei die Packung die Form eines länglichen Streifens (1) hat, der so flexibel ist, dass er zur Einführung in eine Inhalationsvorrichtung zu einer Rolle aufgewickelt werden kann, und eine Basis (2), in der die Blisterhohlräume (4) ausgebildet sind, und einen im Wesentlichen planaren Deckelabschnitt (3) aufweist, der die Blisterhohlräume (4) versiegelt, wobei die Basis (2) und der Deckelabschnitt (3) jeweils ein Laminat umfassen, das eine Folienschicht (2b, 3b), die feuchtigkeits- und gasundurchlässig ist, eine äußere Polymerschicht (2c, 3c) und eine innere Polymerschicht (2a, 3a) auf der der äußeren Polymerschicht (2c, 3c) gegenüberliegenden Seite der Folienschicht (2b, 3b) aufweist, wobei das Verfahren den Schritt aufweist, bei dem ein Abschnitt der äußeren Polymerschicht (2c, 3c) von der Basis oder dem Deckelabschnitt im Wesentlichen entfernt wird oder ein Abschnitt der äußeren Polymerschicht (2c, 3c) der Basis oder des Deckelabschnitts ohne Beschädigung der Folienschicht (2b, 3b) des Laminats verdünnt wird, um den Schwächebereich zu bilden.

15. Verfahren nach Anspruch 14, wobei der Schritt, bei dem ein Abschnitt der äußeren Polymerschicht (2c) zum Bilden des Schwächebereichs (6) vom Basislaminat im Wesentlichen entfernt oder verschoben wird, den Schritt des Beaufschlagens der Packung mit Wärme aufweist, um einen Abschnitt der äußeren Polymerschicht (2c) von der Folienschicht (2b) des Bereichs zu erweichen oder zu schmelzen.

16. Verfahren nach Anspruch 15, einschließlich des Schritts des Ausbildens von Anreißmitteln (13) dort, wo der Schwächebereich (6) auf einen Rand der Packung trifft.

17. Verfahren nach Anspruch 15 oder 16, wobei der Schritt des Bildens des Schwächebereichs (6) durch Beaufschlagen der Packung mit Wärme, um einen Abschnitt der äußeren Polymerschicht (2c) zu erweichen oder zu schmelzen, ohne die Folienschicht (2b) zu beschädigen, den Schritt des Verwendens eines Lasers umfasst.

18. Inhalationsvorrichtung, die eine Blisterpackung nach einem der Ansprüche 1 bis 13 enthält.

## Revendications

1. Emballage alvéolaire sous la forme d'une bandelette allongée qui est suffisamment souple pour pouvoir être enroulée en un rouleau en vue de son insertion dans un dispositif d'inhalation comprenant une pluralité de cavités formant alvéoles (4) espacées qui reçoivent et stockent une dose individuelle de médicament en poudre destiné à être inhalé par un utilisateur, l'emballage comprenant une base (2) dans laquelle sont formées les cavités formant alvéoles (4) et une partie opercule (3) essentiellement plane fermant hermétiquement les cavités formant alvéoles (4), la base (2) et la partie opercule (3) comprenant chacune un stratifié, le stratifié de base comprenant une couche de film (2b, 3b) qui est imperméable à l'humidité et aux gaz, une couche polymère extérieure (2c) et une couche polymère intérieure (2a) sur le côté de la couche de film (2b, 3b) opposé à celui de la couche polymère extérieure (2c), **caractérisé par** une zone de faiblesse (6) formée en retirant essentiellement une partie de la couche polymère extérieure (2c) du stratifié de base, ou en affinant une partie de ladite couche polymère extérieure (2c) du stratifié de base, entre chaque cavité formant alvéole (4), ou un certain nombre de cavités formant alvéoles (4), sans endommager la couche de film (2b, 3b) dudit stratifié de base.

2. Emballage alvéolaire selon la revendication 1, dans lequel la zone de faiblesse (6) est formée en déplaçant essentiellement la couche polymère intérieure (2a), en plus de retirer ou déplacer essentiellement la couche polymère extérieure (2c), vis-à-vis du stratifié de base.

3. Emballage alvéolaire selon la revendication 2, dans lequel la couche polymère intérieure (2a) présente une épaisseur qui est inférieure à 60 µm.

4. Emballage alvéolaire selon la revendication 3, dans lequel la couche polymère intérieure (2a) présente une épaisseur comprise entre 15 µm et 40 µm.

5. Emballage alvéolaire selon la revendication 4, dans lequel la couche polymère intérieure (2a) présente une épaisseur de 30 µm.

6. Emballage alvéolaire selon l'une quelconque des revendications précédentes, dans lequel la zone de faiblesse (6) est présente entre chaque alvéole de la bandelette.

7. Emballage alvéolaire selon l'une quelconque des revendications précédentes, dans lequel la ou chaque zone de faiblesse (6) est discontinue.

8. Emballage alvéolaire selon la revendication 7, dans lequel la couche polymère extérieure (2c) est essentiellement retirée ou déplacée au niveau de régions séparées, espacées, s'étendant le long de chaque zone de faiblesse (6) de telle sorte que chaque zone de faiblesse (6) soit constituée d'une série de sections affaiblies et non-affaiblies.

9. Emballage alvéolaire selon l'une quelconque des revendications précédentes, dans lequel la zone de faiblesse (6) est formée au niveau d'un bord d'un emballage afin de faciliter la formation d'une déchirure.

10. Emballage alvéolaire selon l'une quelconque des revendications précédentes, comprenant une encoche, une entaille ou une perforation (13) dans le bord de l'emballage afin de faciliter la formation d'une déchirure.

11. Emballage alvéolaire selon l'une quelconque des revendications précédentes, dans lequel au moins une partie de la zone de faiblesse (6) est une bandelette étroite ou une ligne.

12. Emballage alvéolaire selon la revendication 11, dans lequel la zone de faiblesse (6) comprend une bandelette étroite ou une ligne et une zone de faiblesse élargie à l'endroit où la ligne rejoint un bord de l'emballage.

13. Emballage alvéolaire selon la revendication 12, dans lequel la bandelette étroite ou la ligne s'étend en travers de l'emballage entre deux zones de faiblesse élargies à l'endroit où les extrémités de la ligne rejoignent les bords de l'emballage.

14. Procédé de formation d'une zone de faiblesse dans un emballage alvéolaire entre des cavités formant alvéoles (4) adjacentes de l'emballage qui reçoivent et stockent des doses individuelles de médicament en poudre destiné à être inhalé par un utilisateur, l'emballage se présentant sous la forme d'une bandelette allongée (1) qui est suffisamment souple pour pouvoir être enroulée en un rouleau en vue de son insertion dans un dispositif d'inhalation et comprenant une base (2) dans laquelle sont formées les cavités formant alvéoles (4) et une partie opercule (3) essentiellement plane fermant hermétiquement les cavités formant alvéoles (4), la base (2) et la partie opercule (3) comprenant chacune un stratifié comprenant une couche de film (2b, 3b) qui est imperméable à l'humidité et aux gaz, une couche polymère extérieure (2c, 3c) et une couche polymère intérieure (2a, 3a) sur le côté de la couche de film (2b, 3b) opposé à celui de la couche polymère extérieure (2c, 3c), le procédé comprenant l'étape consistant à retirer essentiellement une partie de la couche polymère extérieure (2c, 3c) de la partie base ou la partie opercule, ou à affiner une partie de ladite couche polymère extérieure (2c, 3c) de la partie base ou la partie opercule, sans endommager la couche de film (2b, 3b) du stratifié, afin de former ladite zone de faiblesse.

15. Procédé selon la revendication 14, dans lequel l'étape consistant à retirer ou déplacer essentiellement une partie de la couche polymère extérieure (2c) vis-à-vis du stratifié de base afin de former ladite zone de faiblesse (6) comprend l'étape consistant à appliquer de la chaleur à l'emballage afin de ramollir ou de faire fondre une partie de la couche polymère extérieure (2c) vis-à-vis de la couche de film (2b) dans ladite zone.

16. Procédé selon la revendication 15, comprenant l'étape consistant à créer des moyens de formation d'une déchirure (13) à l'endroit où la zone de faiblesse (6) rejoint un bord de l'emballage.

17. Procédé selon la revendication 15 ou 16, dans lequel l'étape consistant à former ladite zone de faiblesse (6) en appliquant de la chaleur à l'emballage afin de ramollir ou de faire fondre une partie de la couche polymère extérieure (2c) sans endommager la couche de film (2b) comprend l'étape consistant à utiliser un laser.

18. Dispositif d'inhalation contenant un emballage alvéolaire selon l'une quelconque des revendications 1 à 13.
